# EUROPEAN PATENT APPLICATION

(11) **EP 1 486 192 A1**
(43) Date of publication of application: **15.12.2004**
(21) Application number: 03710348.8
(22) Date of filing: 13.03.2003
(51) Int. Cl.: A61J 1/00

(54) **HIGH-TEMPERATURE-STERILIZABLE INSTILLATOR**

(30) Priority: 18.03.2002 JP 2002074205
(71) Applicant: Santen Pharmaceutical Co., Ltd, Osaka-shi, Osaka 533-0021 (JP)
(72) Inventor: KUSU, Yukio c/o SANTEN PHARMACEUTICAL CO., LTD., Osaka-shi, Osaka 533-0021 (JP)
(74) Representative: Petersen, Frank, Dipl.-Ing.
(86) International application number: PCT/JP2003/003036
(87) International publication number: WO 2003/077824

(57) **Abstract**

To provide an eyedrops container (A) which is easy to manufacture and easy to sterilize, ensures a sterile guarantee, and moreover is excellent in instilling operability, the eyedrops container (A) is integrally formed of a resin material that does not melt or deform in time of sterilization performed under a condition of 121°C for 20 minutes.

## Description

### TECHNICAL FIELD

This invention relates to a medical eyedrops container (hereinafter simply called an eyedrops container) and an eyedropper for containing medical eyedrops.

### BACKGROUND ART

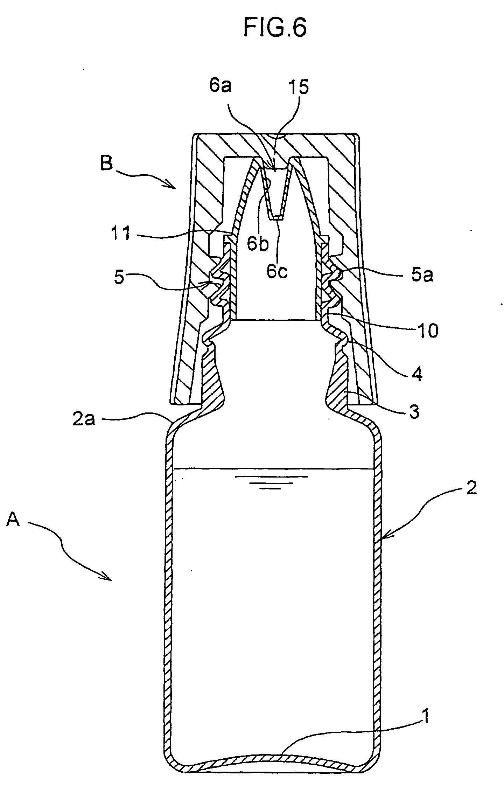

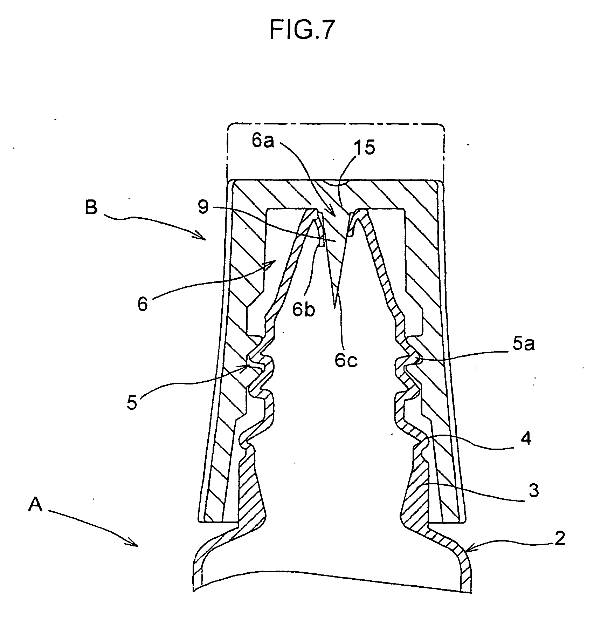

An existing eyedrops container filled up with medical eyedrops is as shown in Fig. 6 or 7, for example. What is called a three-piece type eyedrops container (Fig. 6) including a main container body 10 in the form of a hollow cylinder, with a stopper 11 mounted thereon, and with a cap included to make three components forming a whole eyedropper, and what is called a bottle pack eyedrops container which may be an integral molding type container (Fig. 7) with an instilling tube and a main container body formed integrally by blow molding technique are in wide use. This bottle pack eyedrops container is filled up with a predetermined quantity of medical fluid and sealed simultaneously with a blow molding or vacuum forming process. The container of Fig. 7, in particular, has a construction in which, to a male screw 5a of a flexible main container body A formed of a thermoplastic material, a cap B having a needle-like projection 9 formed integral therewith for perforating and forming an instilling bore in a tip end of the main container body A is removably screwed. This container forms an instilling bore 6c in an instilling portion at the tip end of the main container body A with the needle-like projection 9 of the cap B by screwing the cap B to a deeper screw-in position than a normal closing position. As a further eyedrops container, what is called an open bottle pack eyedrops container has been considered as shown in Japanese Patent Application Laid-open No. 2001-120639, with the tip end of the above bottle pack eyedrops container made into a tip configuration having a stopper function, and an instilling bore 6c formed there in advance.

Since eyedrops are applied directly to the eye which is an especially sensitive organ in the human body, it is strictly required to maintain them in an aseptic condition until instillation. Therefore, the above three-piece type eyedrops container, for example, is made by filling it with a medical fluid after sterilizing each component with EO gas, electron beams or gamma rays.

On the other hand, the bottle pack eyedrops container or open bottle pack eyedrops container is integrally molded under heated condition by the blow molding method or the like. Thus, a medical fluid may be filled without requiring sterilization of the container.

However, in either case, the medical fluid needs to be filled into the eyedrops container in a sterilized state. Thus, after sterilizing it with steam sterilization or sterilization by filtration using a membrane filter and so on, an aseptic filling is carried out in an aseptic room meeting strict conditions. However, according to such a filling method, a filling operation is complicated and working efficiency becomes low, and moreover a massive equipment investment is needed.

With regard to the above technique, if the whole eyedrops container can be heat-sterilized after being filled with the medical fluid, the sterilizing operation can be carried out all at once and the aseptic filling will no longer be required. In this case, the massive equipment investment noted above can also be omitted. Conventionally, a careful inspection also is required of the sterile condition for every sterilizing operation. Where heat sterilization is performed all at once, such an inspecting operation may be carried out only after a final sterilizing operation, and this improves working efficiency.

Then, researches have been made over many years for a technique of sterilizing the eyedrops container together with the medical fluid, but have not achieved success yet. An eyedrops container enabling such a technique is desired.

In the three-piece type eyedrops container noted hereinbefore, the main container body and stopper, generally, are formed of resins different in the quality of material. That is, the main container body is formed of a soft resin in order to facilitate discharge of the medical fluid. The stopper is formed from of a relatively hard resin in order to ensure discharge of the medical fluid in a fixed quantity.

Thus, the main container body and stopper are formed of different materials, and the resin materials have different coefficients of heat contraction. Heat sterilization carried out after assembling them together will make it difficult to secure liquid-tightness, with the stopper falling off the main container body, or joints of the assembly becoming loose. Where the main container body and stopper are formed of a relatively hard material, the accuracy of dimensions presents a problem in time of fitting and assembling the two, again tending to pose a problem in securing liquid-tightness. Further, where the main container body is hard, a strong force is needed to discharge the medical fluid, which is a factor to lower operability.

On the other hand, an integral molding, such as the bottle pack eyedrops container or open bottle pack eyedrops container, is free from the problem of falling off the stopper since there is no stopper. It is usually made of a soft resin in order to facilitate discharge of the medical fluid and to maintain molding efficiency.

However, the container formed of a soft resin tends to undergo a melting deformation in time of heat sterilization. This therefore poses a problem of being unfit for medical or other use, with the shape of instilling bore 6c becoming unstable to vary the quantity of medical fluid discharged.

Having regard to the drawbacks noted above, the object of this invention, therefore, is to provide an eyedrops container which is easy to manufacture and easy to sterilize, ensures a sterile guarantee, and moreover is excellent in instilling operability.

### DISCLOSURE OF THE INVENTION

Inventors have noted that all bacilli become extinct when heated to 121°C for 20 minutes, and carried out intensive research. As a result, this invention has been conceived on the assumption that a desired eyedrops container may be obtained by forming it integrally by using a resin material that withstands the above condition and is free from melting and deformation.

An eyedrops container according to this invention is characterized in that it is integrally formed of a resin material that does not substantially melt or deform in time of sterilization performed under a condition of 121°C for 20 minutes.

Preferably, said resin material is polypropylene-polyethylene random copolymer.

Further, said polypropylene polyethylene-random copolymer contains 1 to 10%, particularly 3 to 5%, of polyethylene.

Preferably, hollows are formed on a flexible barrel portion in form of a hollow cylinder to be capable of being held by tips of two digits.

Further, an eyedropper using such an eyedrops container for medical use, with a cap for screwing thereto,
is characterized in that a bottomed conical recess having an inside diameter increased toward a tip end is formed in an instilling tube formed on a tip region of said eyedrops container for medical use, the recess having an instilling portion formed in a bottom thereof.

As noted hereinbefore, the conventional eyedrops container requires the main container body and stopper, for example, to be sterilized separately and assembled under sterile conditions, with a sterilized medical fluid filled aseptically. For that matter, since the eyedrops container according to this invention is integrally formed of a resin material that does not substantially melt or deform in time of sterilization performed under a condition of 121°C for 20 minutes, it may be sterilized under the condition of 121°C for 20 minutes after being filled with a medical fluid. Thus, the conventional complicated process can now be completed in one sterilizing operation after making the eyedrops container and filling it with a medical fluid, thereby achieving simplification of the manufacture process. Such a sterilizing process is a strict sterilizing process said to kill all ordinary bacteria, and therefore a sterile guarantee can be ensured by one sterilizing operation.

Where said resin material is polypropylene-polyethylene random copolymer, not only its heat resistance is high and the above sterilizing process is possible, but since it is material soft enough to allow a relatively easy instilling operation, though it is a hard material, its processability is high, and the eyedrops container having the main container body and stopper formed integrally can be manufactured with sufficient workability, and instilling operability also is maintained excellent.

Further, where said polypropylene polyethylene-random copolymer contains 1 to 10%, particularly 3 to 5%, of polyethylene, the resin material has hardness to realize the processability and instilling workability at the same time without spoiling the above heat resistance.

Where hollows are formed on a flexible barrel portion in form of a hollow cylinder to be capable of being held by tips of two digits, the hollows formed on the barrel portion of the container is gripped by the tips of two digits when applying the medical fluid in the container. Thus, stable holding positions are provided for the digit-tips. Moreover, when pressing the barrel portion of the container, the hollows already formed in the parts of the barrel portion in contact with the digit-tips can reduce the pressing force, compared with the case of depressing and deforming parts of the barrel portion in the form of a cylinder against its elastic restoring force.

Thus, though this is a simple and inexpensive reconstruction for forming hollows in the barrel portion in the form of a hollow cylinder, it is easy to hold, compared with the conventional eyedrops container. In addition, since instilling operability is improved by a reduction in the pressing force, the medical fluid in the container may be applied accurately and easily.

Further, where an eyedropper using said eyedrops container has a cap for screwing to the eyedrops container, switching may be made between an attached state where the cap is screwed to the eyedrops container for sanitarily storage, and a detached state where the cap is removed to allow an instilling operation.

A bottomed conical recess having an inside diameter increased toward a tip end is formed in the instilling tube formed on the tip region of said eyedrops container for medical use. The recess has an instilling portion formed in the bottom thereof to act as a stopper. The quantity of one drop in time of instillation may be adjusted uniform.

### BRIEF DESCRIPTION OF THE DRAWINGS

Fig. 1 is a view showing an outline of an eyedropper according to this invention;
Fig. 2 is a front sectional view of a tip portion of an eyedrops container, with a cap and the eyedrops container separated;
Fig. 3 is a front sectional view of the tip portion of the eyedrops container, with the cap and the eyedrops container screwed together;
Fig. 4 is an overall side view of the eyedrops container;
Fig. 5 is a view showing a use state in time of distillation;
Fig. 6 is a view showing an eyedropper having an eyedrops container with a stopper;
Fig. 7 is a sectional side view of an eyedrops container penetrable to form an instilling bore;
Fig. 8 is a view showing a process of blow molding or vacuum forming of the eyedrops container; and
Fig. 9 is an explanatory view showing a method of manufacturing the eyedropper.

### BEST MODE FOR CARRYING OUT THE INVENTION

An embodiment of this invention will be described hereinafter with reference to the drawings.

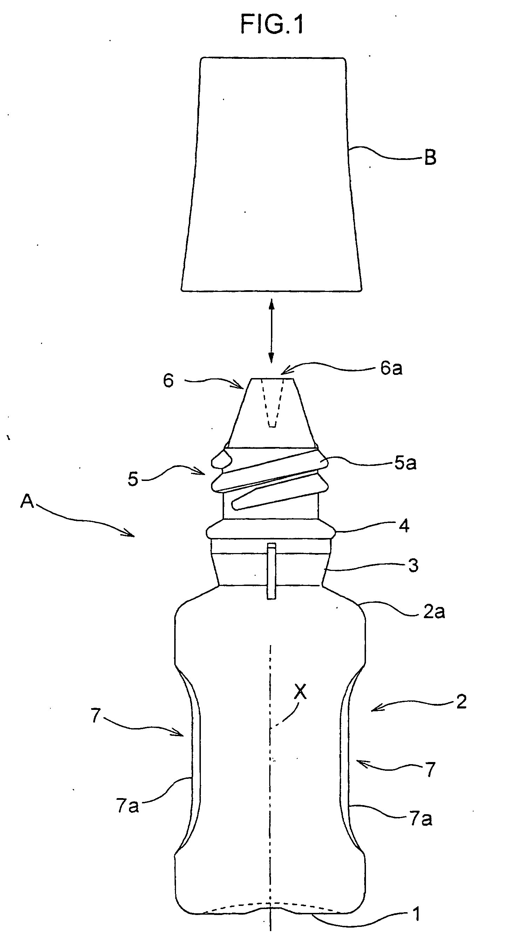

Figs. 1-5 show an eyedropper according to this invention which is mainly used for medical application. This eyedropper includes a flexible eyedrops container A formed of a thermoplastic material and filled with a predetermined quantity of medical fluid simultaneously with a blow molding or vacuum forming process, and a cap B removably screwed to the male screw 5a formed on the threaded tube 5 of the eyedrops container A.

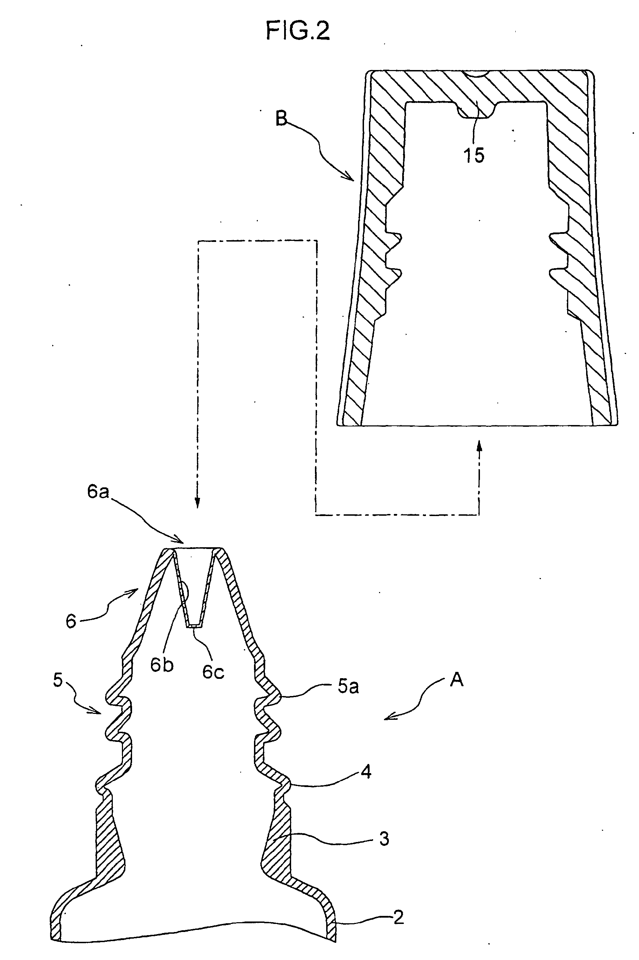

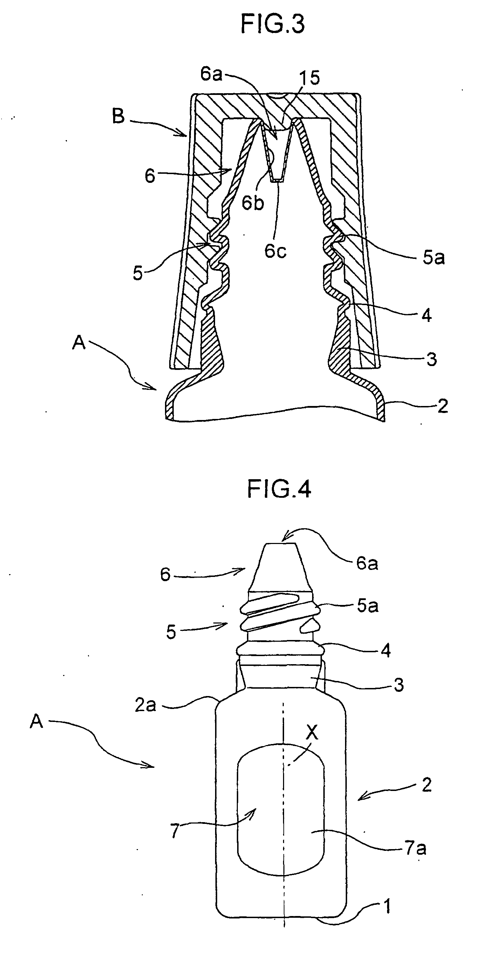

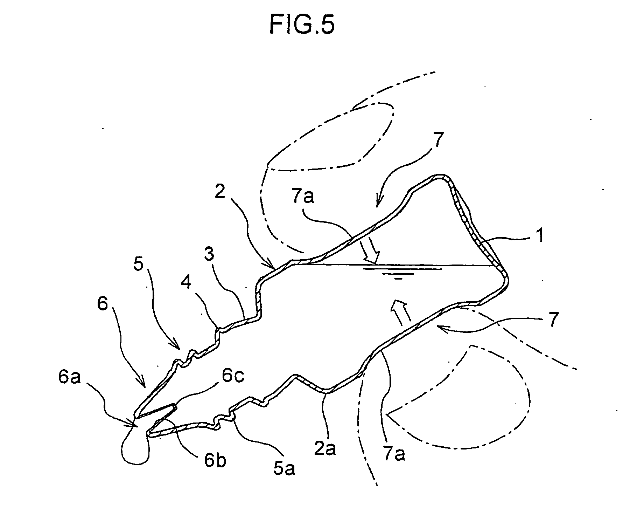

Fig. 1 is a view showing an outline of the eyedropper according to this invention. Fig. 2 is a front sectional view of a tip portion of the eyedrops container, with the cap and the eyedrops container separated. Fig. 3 is a front sectional view of the tip portion of the eyedrops container, with the cap and the eyedrops container screwed together. Fig. 4 is an overall side view of the eyedrops container. Fig. 5 is a view showing a use state in time of distillation.

The eyedrops container A includes a circular bottom 1 curved inward, a barrel portion 2 in the form of a hollow cylinder continuous from edges thereof, a cylindrical neck 3 continuous from a shoulder 2a of the barrel portion 2, an annular step 4 bulging diametrically outward from the top of the neck 3, the threaded tube 5 continuous upward therefrom and having the male screw 5a, and an instilling tube 6 continuous upward therefrom and having an instilling opening 6a.

The eyedrops container A is formed of a resin material that does not melt or deform in time of sterilization performed under the condition of 121°C for 20 minutes, which may be polypropylene-polyethylene random copolymer, polypropylene or the like. The entire container A formed is elastically deformable.

As these material characteristics, MI may be cited. Preferred MI is 0.2 to 2.5, and more preferably 0.3 to 1.5.

As polypropylene-polyethylene random copolymer, a random polymer of propylene containing 1 to 10%, particularly 3 to 5%, of ethylene is applicable, which has sufficient forming processability, and well withstands the above sterilizing condition, and thus suitable for manufacture of eyedrops containers.

When polypropylene is applied, it well withstands the above sterilizing condition, but may present difficulties in forming eyedrops containers of certain shapes, and thus suitable for forming eyedrops containers of simple shape.

As shown in Fig. 2, the instilling tube 6 of the eyedrops container A has a bottomed conical recess 6b formed therein with an inside diameter progressively increasing toward the instilling opening 6a (i.e. toward the tip). A small-diameter instilling bore 6c is formed in an instilling position formed in the bottom of this recess 6b.

The depth of the recess 6b is in the range of 2 to 7mm, desirably in the range of 5 to 7mm, and most desirably 6mm. The bore diameter (opening diameter) of the instilling opening 6a is adjusted to the range of f2mm to f4mm according to the property of the medical fluid.

In order to fix the quantity of one drop (to adjust the quantity of one drop to be within the range of 25 to 50µL according to purpose), the bore diameter of the instilling opening 6a is reduced where the fluid has high surface tension, and the bore diameter of the instilling opening 6a is enlarged where the fluid has low surface tension.

Further, the instilling bore 6c is formed by using a needle with a diameter in the range of f0.1mm to f0.8mm. The smaller the diameter of the needle is, the better, and the most desirable is about f0.2mm. An excessively small diameter causes a technically difficult situation, and therefore a needle in the range of f0.4mm to f0.6mm is used in practice.

Thus, by appropriately setting the bore diameter of the instilling opening 6a, the depth of the recess 6b, and further the pore diameter of the instilling bore 6c, the quantity of drops pushed out of the eyedrops container A by a finger pressure applied to the barrel portion 2 may be controlled to a set quantity.

As shown in Figs. 1 and 4, the barrel portion 2 of eyedrops container A has hollows 7 formed thereon that can be held by tips of two digits. These hollows 7 are in the form of a pair of flat or approximately flat pinching surfaces 7a formed as depressed in two positions circumferentially of the barrel portion 2, and opposed to each other across an axis X of the container.

Each of the pinching surfaces 7a is gently curved with a curvature smaller than the curvature of other parts of the barrel portion 2 as seen in the direction of the container axis X, and formed to have an intermediate portion excluding opposite end portions in the direction of the container axis X extending straight parallel to the container axis X as seen radially (seen from the front) perpendicular to the direction of the container axis X. Of course, this surface may be curved as a whole.

The cap B has a first sealing projection 15 formed integrally therewith for fitting in and sealing the recess 6b of the eyedrops container A when the cap B is screwed to the male screw 5a of the eyedrops container A.

The amount of projection of the first sealing projection 15 is about 0.6 to 1.0mm.

With the above construction, the cap B may be screwed effectively to the eyedrops container A to be joined together.

The material of this cap B is polypropylene-polyethylene random copolymer.

### [Method of Manufacturing the Eyedropper]

A method of manufacturing the eyedropper in this application will be described hereinafter, in the order of manufacture of the eyedrops container and integration with the cap.

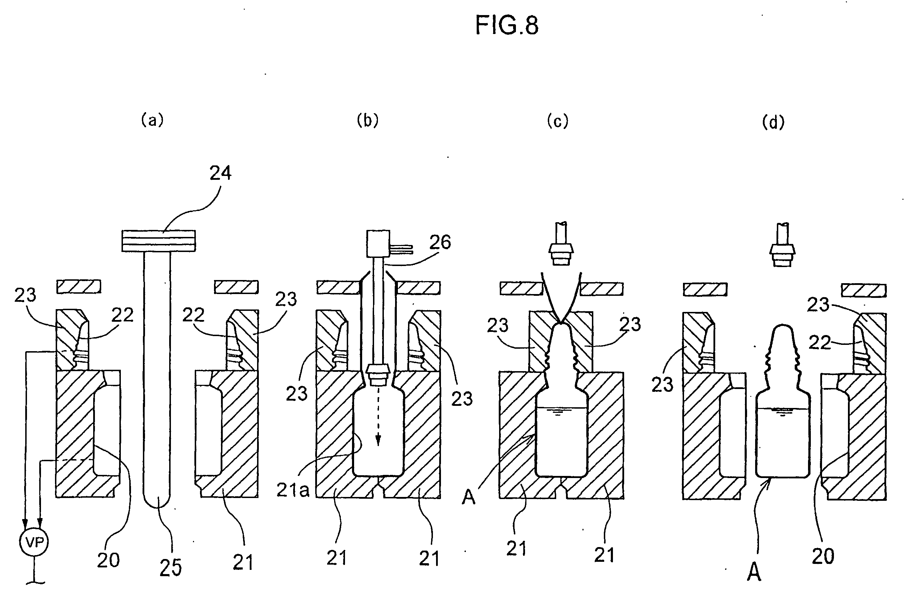

The method of manufacturing the eyedrops container A before the recess 6b and instilling bore 6c are formed is well known in the technical field concerned, and will therefore be described briefly with reference to Fig. 8.

As shown in Fig. 8(a), a pair of main forming dies 21 having a first cavity 20 for forming a portion ranging from the annular step 4 to the bottom 1 of the eyedrops container A, and a pair of auxiliary forming dies 23 having a second cavity 22 for forming the threaded tube 5 and instilling tube 6 of the eyedrops container A, are opened and, from an extruding head 24 disposed above, a barison 25 having a predetermined length which is a half-melted thermoplastic material in the form of a long and thin hollow tube is pushed out vertically through a space between the dies 21 and 23.

Next, as shown in Fig. 8(b), the main forming dies 21 are closed, and the barison 25 is expanded and shaped along die surfaces 21a of the main forming dies 21 by compressed air blow-in action or vacuum action. In this state, as shown in Fig. 8(b), a liquid (medical fluid) is filled in a predetermined quantity from a medicine supply pipe 26.

After this liquid filling step, as shown in Fig. 8(c), the auxiliary forming dies 23 are closed, and the barison 25 is expanded and shaped along die surfaces of the auxiliary forming dies 23 by compressed air blow-in action or vacuum action. Simultaneously with this formation, the liquid filled in is sealed (encapsulated). Then, the step of Fig. 8(d) is executed to complete the operation.

Next, a manufacturing method will be described, for forming the bottomed conical recess 6b and small instilling bore 6c in the instilling tube 6 forming the tip end of the eyedrops container A blow-molded or vacuum-molded as described above.

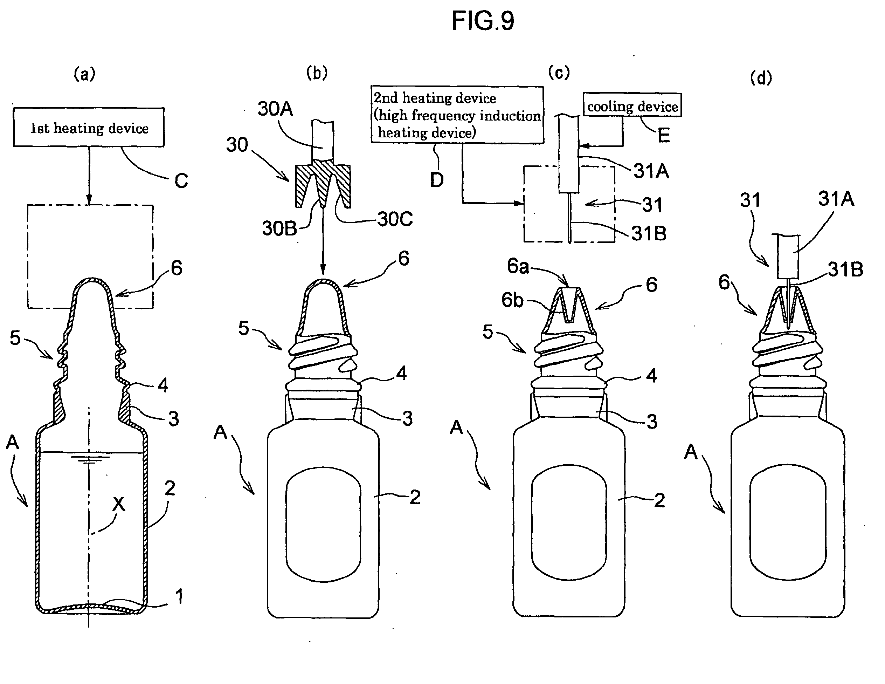

A manufacturing method of a first mode shown in Figs. 9 (a) - (d) uses a projecting metallic forming die 30 for forming the bottomed conical recess 6b, and a needle-like metallic forming die 31 for forming the instilling bore 6c.

The projecting forming die 30 includes, formed on a distal end of a mounting shaft 30A, a conical forming projection 30B for forming the bottomed conical recess 6b, and a bowl-shaped (bell-shaped) die surface 30C for forming an outer peripheral surface of the instilling tube 6 of the eyedrops container A. The needle-like forming die 31 has a needle-like forming projection 31B formed on a distal end of a mounting shaft 31A for forming the small instilling bore.

In the manufacturing method of the first mode, as shown in Fig. 9(a), part of the instilling tube 6 at the tip end of the eyedrops container A is heated to room temperature or 70°C to 150°C by a first heating device C such as warm air, a halogen lamp or laser beams. Although it depends on the material and shape of the eyedrops container A, the heating temperature preferably is a temperature for slightly softening the tip end of the eyedrops container A.

Where the thermoplastic material of the eyedrops container A is a soft resin material such as polyethylene, the tip end will buckle unless heated. It is therefore necessary to heat, with the first heating device C, the part to be shaped by the projecting forming die 30 at least to a buckle-avoiding temperature before a shaping process. However, in the case of a resin material shape that withstands buckling, i.e. capable of withstanding a pressure of the projecting forming die 30 applied in the direction of the container axis X, the shaping may be performed even at room temperature.

Next, as shown in Fig. 9(b), the projecting forming die 30 is pressed in the direction of the container axis X before the part of the instilling tube 6 of the eyedrops container A heated by the first heating device C cools down, thereby forming, in the instilling tube 6 of the eyedrops container A, the bottomed conical recess 6b with the inside diameter increasing toward the instilling bore 6a.

At this time, the bowl-shaped die surface 30C of the projecting forming die 30 can remove burrs formed in time of blow molding and projecting from the outer peripheral surface of the instilling tube 6 of the eyedrops container A.

The projecting forming die 30 itself carries out a temperature control in a range from room temperature to 150°C in accordance with the shape and wall thickness of the instilling tube 6 of the eyedrops container A to be molded. The heating temperature, preferably, is the lowest possible temperature, taking into consideration a cooling solidification at the tip end of the instilling tube 6.

The projecting forming die 30 is arranged readily changeable according to the property of the liquid to be filled.

Next, as shown in Figs. 9 (c) and (d), the needle-like forming die 31 is pressed in the direction of the container axis X on the central position in the bottom of the recess 6b formed in the instilling tube 6 of the eyedrops container A. Thus, a small instilling bore is formed to be capable of controlling to a set quantity the quantity of droplets pushed out of the eyedrops container A as the barrel portion 2 is pressed by tips of digits.

In the step of forming the instilling bore 6c by the needle-like projection 31B of this needle-like forming die 31, a method of operating the needle-like projection 31B at room temperature, and a method of operating the needlelike projection 31B after heating it, are proposed. The method to be adopted is selected according to conditions such as the shape of the instilling bore 6c to be formed, the shape of recess 6b, further the shapes and materials of the container and others, manufacturing cost and so on. As the heating temperature, where heating is required, it is advantageous to heat at least the needle-like projection 31B of the needle-like forming die 31 to a temperature for fusing the resin material of the container, i.e. to a range of 130°C to 180°C.

A second heating device D such as high frequency induction heating, a halogen lamp or warm air is used to heat the needle-like forming die 31. The mounting shaft 31A acting as the root of the needle-like forming die 31 is constructed to be cooled by a cooling device E such as a water jacket, compressed air or the like.

At a point of time when the needle-like forming die 31 has been cooled to a predetermined temperature, the needle-like forming die 31 is extracted along the direction of the container axis X from the instilling tube 6 of the eyedrops container A molded to the predetermined shape.

The needle-like forming die 31 may be given a surface treatment such as plating, fluororesin coating, or special plating in order to improve separabiltiy and releasability of the resin. This surface treatment, preferably, is what can withstand high temperature and does not separate easily.

While the main container body A may be obtained as described above, the cap B used in this application is formed and obtained beforehand in the predetermined shape so far described.

Now, after the manufacture of the main container body A is completed as described above, the cap is initially attached to the main container body A, i.e. for the first time. At this time, the cap B is screwed to the main container body A in a state that the thermoplastic material of the main container body A retains plasticity, and a certain degree of plasticity so that an outline configuration of the main container body A does not collapse as a result of screwing of the cap B.

At this time, since the construction provided with the first sealing projection 15 is adopted for the cap B, the first sealing projection 15 is placed in tight contact with the recess 6b to secure an airtight condition.

With the above construction is adopted, the eyedrops container obtained has an excellent sealing performance and is convenient in use.

### [Embodiment]

The eyedrops container with a capacity of 5ml has been obtained under various molding conditions and sterilizing temperature conditions according to the above embodiment. The hollows 7 provide medical fluid press-out performances as set out in Table 1.

**Table 1**

| | PP resin | sterilizing conditions | total amount of resin | average pressing force (N) |
|---|---|---|---|---|
| 1 | PP random copolymer (Sun Allomer PB222A) contains 4% of PE | 121°C 20min. | 2.2g | 18.73 |
| 2 | | | 2.0g | 13.82 |
| 3 | | | 1.8g | 12.11 |
| 4 | | 115°C 20min. | 2.2g | 17.79 |
| 5 | | | 2.0g | 13.23 |
| 6 | | | 1.8g | 11.30 |
| 7 | | 110°C 20min. | 2.2g | 18.17 |
| 8 | | | 2.0g | 13.08 |
| 9 | | | 1.8g | 10.87 |
| 10 | | 105°C 20min. | 2.2g | 17.09 |
| 11 | | | 2.0g | 13.22 |
| 12 | | | 1.8g | 12.54 |
| 13 | PP random copolymer (Novatec PP EG8) contains 3% of PE | 121°C 20min. | 2.2g | 14.96 |
| 14 | | | 2.0g | 13.90 |
| 15 | | | 1.8g | 11.09 |
| 16 | | 115°C 20min. | 2.2g | 14.58 |
| 17 | | | 2.0g | 14.95 |
| 18 | | | 1.8g | 10.64 |
| 19 | | 110°C 20min. | 2.2g | 15.31 |
| 20 | | | 2.0g | 14.87 |
| 21 | | | 1.8g | 11.45 |
| 22 | | 105°C 20min. | 2.2g | 15.68 |
| 23 | | | 2.0g | 14.06 |
| 24 | | | 1.8g | 10.36 |

From this table, it is seen that, when the eyedrops container is molded integrally by using the above resin material, what withstands a sterilizing operation at 121°C for 20 minutes can be molded easily even when the 5ml container is formed of 1.8g resin, and that medical fluid press-out operability in that case may also be set to a good value of about 10 to 12N. The press-out operability is measured as follows.

The eyedrops container A containing water is held, with the instilling tube 6 facing down, and to be capable of pressing central parts of outer side surfaces of the hollows 7 of the eyedrops container A. After confirming that the inside except the recess 6b of the instilling tube 6 is not filled with the water (that air is not trapped adjacent the instilling opening 6a), the spacing with which the eyedrops container held is narrowed and the hollows 7 are pressed. A pressing force needed for one drop of the water to fall from the instilling opening 6a of the eyedrops container made as noted above is measured with a digital force gauge.

Regarding the press-out force, it is preferred to set it to 15N or less since the user will feel a strong resistance against the digit-tips in time of instillation operation if the press-out force reaches or exceeds 20N. An eyedrops container has been formed of the same resin material as above, that withstands the same sterilizing conditions and has no hollows. This results in a press-out force of about 35.3N (3.64kgf). The product according this invention is extremely excellent in instilling operability.

### [Other Embodiments]

In each of the above embodiments, the eyedrops container has been described, in which the bottomed conical recess 6b with the inside diameter enlarging toward the instilling opening 6a, and the small instilling bore 6c capable of controlling to a set quantity the quantity of droplets pushed out of the eyedrops container A as the barrel portion 2 is pressed by tips of digits, are formed beforehand in the instilling tube 6 of the main container body A blow-molded or vacuum formed. The present invention is not limited to such an eyedrops container. As shown in Fig. 6, a cap B having a needle-like projection 9 formed integrally therewith for penetrating and forming an instilling bore in a tip end of a main container body A may be removably screwed to a male screw 5a of a flexible main container body A formed of a thermoplastic material with a predetermined quantity of medical fluid filled and sealed simultaneously with a blow molding or vacuum forming process. An instilling bore 6c is formed in an instilling portion at the tip end of the main container body A with the needle-like projection 9 of cap B by a screwing operation to a deeper screw-in position than a normal closing position of the cap B.

The other aspects of the construction are the same as in the construction described in the embodiment. Like numerals are used to identify like parts which are the same as in the embodiment and will not be described again.

In the foregoing embodiment, the eyedrops container with hollows has been described. The present invention is not limited to this shape, but is applicable also to one having no hollows formed therein. Further, not only of the cylindrical shape, but various shapes may be employed such as elliptical, which are set based on the resin material used, application and so on.

### INDUSTRIAL AVAILABILITY

An eyedrops container which is easy to manufacture and easy to sterilize, ensures a sterile guarantee, and moreover is excellent in instilling operability may be provided.

## Claims

1. An eyedrops container for medical use integrally formed of a resin material that does not melt or deform in time of sterilization performed under a condition of 121°C for 20 minutes.

2. An eyedrops container for medical use as defined in claim 1, wherein said resin material is polypropylene-polyethylene random copolymer.

3. An eyedrops container for medical use as defined in claim 2 wherein said polypropylene polyethylene-random copolymer contains 3 to 5% of polyethylene.

4. An eyedrops container for medical use as defined in claim 1 wherein hollows are formed on a flexible barrel portion in form of a hollow cylinder to be capable of being held by tips of two digits.

5. An eyedropper having an eyedrops container A as defined in any one of claims 1 to 4, with a cap B for screwing thereto,
wherein a bottomed conical recess (6b) having an inside diameter increased toward a tip end (6a) is formed in an instilling tube (6) formed on a tip region of said eyedrops container for medical use, the recess (6b) having an instilling portion formed in a bottom thereof.
